# EUROPEAN PATENT SPECIFICATION

(11) **EP 2 976 953 B1**
(45) Date of publication and mention of the grant of the patent: **09.05.2018**
(21) Application number: 13879019.1
(22) Date of filing: 21.03.2013
(51) Int. Cl.: A24F 47/00, A61M 15/06

(54) **DISASSEMBLY-PROOF ATOMIZER AND ELECTRONIC CIGARETTE**
ZERSETZUNGSBESTÄNDIGER ZERSTÄUBER UND ELEKTRONISCHE ZIGARETTE
ATOMISEUR INDÉMONTABLE ET CIGARETTE ÉLECTRONIQUE

(43) Date of publication of application: 27.01.2016
(73) Proprietor: Huizhou Kimree Technology Co., Ltd. Shenzhen Branch, Shenzhen, 518040 (CN)
(72) Inventor: LIU, Qiuming, Shenzhen, Guangdong 518000 (CN)
(74) Representative: Puschmann Borchert Bardehle Patentanwälte Partnerschaft mbB
(86) International application number: PCT/CN2013/073015
(87) International publication number: WO 2014/146284

(56) References cited:
- WO-A1-2008/130813
- WO-A1-2011/124033
- WO-A2-2012/142293
- CN-A- 1 270 130
- CN-A- 101 606 758
- CN-A- 101 843 368
- CN-U- 201 869 778
- CN-U- 202 233 005
- CN-U- 202 233 005
- CN-U- 202 407 083
- US-A1- 2008 177 246
- US-A1- 2012 145 169
- US-A1- 2012 285 475

## Description

### FIELD OF THE INVENTION

The present invention relates to daily electronic products, and more particularly to a disassembly-proof atomizer and an electronic cigarette.

### BACKGROUND OF THE INVENTION

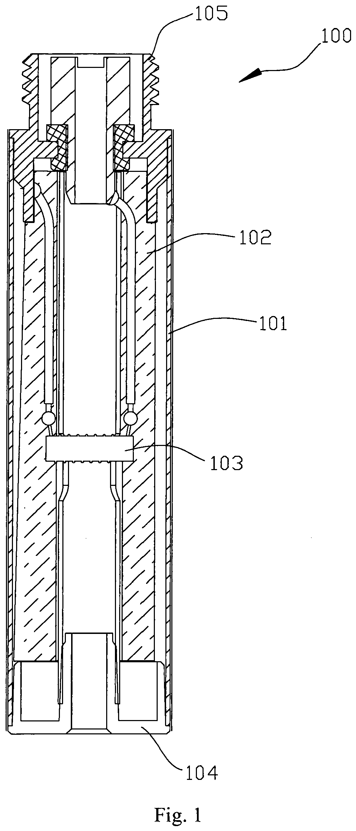

The traditional cigarette is replaced by the electronic cigarette gradually with the improvement in the health consciousness. A disposable electronic cigarette in the prior art comprises an atomizer and a battery cartridge connected with each other. Fig.1 is a schematic diagram showing the atomizer 100 of the disposable electronic cigarette in the prior art. As shown in Fig.1, the atomizer 100 comprises a protective sleeve 101, and an oil-storage cotton 102 and a heating component 103 which are arranged in the protective sleeve. A battery and a controller are arranged in the battery cartridge (not shown), and the heating component 103 is electrically connected with the battery. A suction nozzle cap 104 is arranged on a first end of the atomizer 100. The heating component 103 is electrified and emits heat when the smoker smokes, so as to atomize the cigarette liquid in the oil-storage cotton 102 and form smoke for the smoker's inhalation.

A connector 105 is arranged on a second end of the atomizer 100, the lower part of the connector 105 is fixed in the protective sleeve 101 through an interference fit, and the upper part of the connector 105 extends out from the protective sleeve 101 and connects with the battery cartridge through a screwed connection. When the disposable electronic cigarette is used up, the atomizer 100 is unscrewed from the battery cartridge and is replaced by a new atomizer 100 so that the disposable electronic cigarette can be reused.

The defect of this atomizer is that, when an force is applied on the connector 105 in a direction perpendicular to the protective sleeve 101 to bend the connector, the connector 105 can be disassembled from the protective sleeve 101 easily, and then the oil-storage cotton 102 can be taken out to add unqualified cigarette oil therein for reuse. The unqualified cigarette contains a lot of harmful substance and will damage the smoker's health.

### SUMMARY OF THE INVENTION

Aiming at the defect of the atomizer being disassembled easily in the prior art, the technical problem to be solved by the present invention is to provide a disassembly-proof atomizer and an electronic cigarette so as to prevent the atomizer from being disassembled.

The present invention is as defined by the appended claims and adopts the following technical solution to solve its technical problem: an disassembly-proof atomizer is provided, comprising a protective sleeve, a suction nozzle cap fixed on a first end of the protective sleeve, and a battery cartridge connector fixed on a second end of the protective sleeve; a vent pipe, a heating component and an oil-storage cotton coated outside the vent pipe and the heating component are arranged in the protective sleeve, the battery cartridge connector comprises a protective sleeve connecting part and a battery cartridge connecting part connected with each other, a center hole extending along an axis of the protective sleeve is defined in the battery cartridge connector, and a groove is defined on a joint between the protective sleeve connecting part and the battery cartridge connecting part.

According to the disassembly-proof atomizer of the present invention, the battery cartridge connector has a wall thickness of 0.05-0.2mm at the groove.

According to the disassembly-proof atomizer of the present invention, the protective sleeve is in cylindric shape, and a peripheral of a cross section of the battery cartridge connector at the direction perpendicular to an axis of the protective sleeve is in quadrate, circular, triangular, cone, trapezoidal or wavy shape.

According to the disassembly-proof atomizer of the present invention, a peripheral surface of a cross section of the battery cartridge connector at the groove in a direction perpendicular to an axis of the protective sleeve comprises multiple recesses arranged alternately, and a bulge is formed between every two adjacent recesses.

According to the disassembly-proof atomizer of the present invention, the battery cartridge connector has a wall thickness of 0.05-0.1mm at the recesses and a wall thickness of 0.1-0.2mm at the bulge.

According to the disassembly-proof atomizer of the present invention, the protective sleeve connecting part is fixed in the protective sleeve by interference fit.

According to the disassembly-proof atomizer of the present invention, a chamfer for assisting inserting into the protective sleeve is formed on one end of protective sleeve connecting part, which is away from the battery cartridge connecting part.

According to the disassembly-proof atomizer of the present invention, an external thread is formed on the battery cartridge connecting part.

According to the disassembly-proof atomizer of the present invention, the disassembly-proof atomizer further comprises a first insulating ring fixed in the battery cartridge connector and a first electrode fixed in the first insulating ring, and the first insulating ring is made of elastic material; a first retaining groove is formed on a peripheral surface of the first insulating ring, a first protrudent ring extending along a radial direction of the protective sleeve is formed in the protective sleeve connecting part, and the first retaining groove is engaged with the first protrudent ring.

According to the disassembly-proof atomizer of the present invention, the suction nozzle cap comprises a cover plate with a center hole, an outer cylinder wall extending perpendicularly from the cover plate, and an inner cylinder wall extending perpendicularly from a periphery of the center hole on the cover plate; the outer cylinder wall is fixed in the first end of the protective sleeve through interference fit.

According to the disassembly-proof atomizer of the present invention, the oil-storage cotton is in cylindric shape, with one end abutted against an end of the outer cylinder wall of the suction nozzle cap and the other end sleeved by the protective sleeve connecting part of the battery cartridge connector.

An electronic cigarette is further provided and the electronic cigarette comprises a battery cartridge and the disassembly-proof atomizer described above, and the battery cartridge and the disassembly-proof atomizer connect with each other.

When implementing the disassembly-proof atomizer and electronic cigarette of the present invention, the following advantageous effect can be achieved: the groove is formed on the battery cartridge connector, so that the wall at the groove is very thin and the battery cartridge connector will be broken at the groove when a force is a applied on the battery cartridge connector in the direction perpendicular to the protective sleeve to bend the battery cartridge connector. Thus, the battery cartridge connector will not be disassembled, and the disassembly-proof object is achieved. Accordingly, the unqualified cigarette oil can be effectively prevented from being added into the atomizer of the disposable electronic cigarette and reuse of the atomizer is avoided.

### BRIEF DESCRIPTION OF THE DRAWINGS

The present invention will be described in detail with reference to the accompanying drawings and embodiments, in the accompanying drawings:
Fig. 1 is a schematic diagram showing an atomizer of a disposable electronic cigarette in the prior art;
Fig. 2 is a schematic explosive view showing an atomizer of the present invention;
Fig. 3 is a schematic diagram showing an atomizer of the present invention;
Fig. 4 is a sectional view of the atomizer shown in Fig. 3;
Fig. 5 is a sectional view showing a battery cartridge of the present invention;
Fig. 6 is a schematic diagram showing a battery cartridge connector of the present invention;
Fig. 7 is a sectional view showing the battery cartridge connector of the present invention;
Fig. 8 is a cross section view of the battery cartridge connector, cut at a groove.

### DETAILED DESCRIPTION OF THE EMBODIMENT

The objective, technical scheme and advantages of the present invention will be more apparent from the following detailed description of the present invention with reference to the accompanying drawings and embodiments. It should be understand that the embodiment is described herein to illustrate the present invention, and is not to limit the present invention.

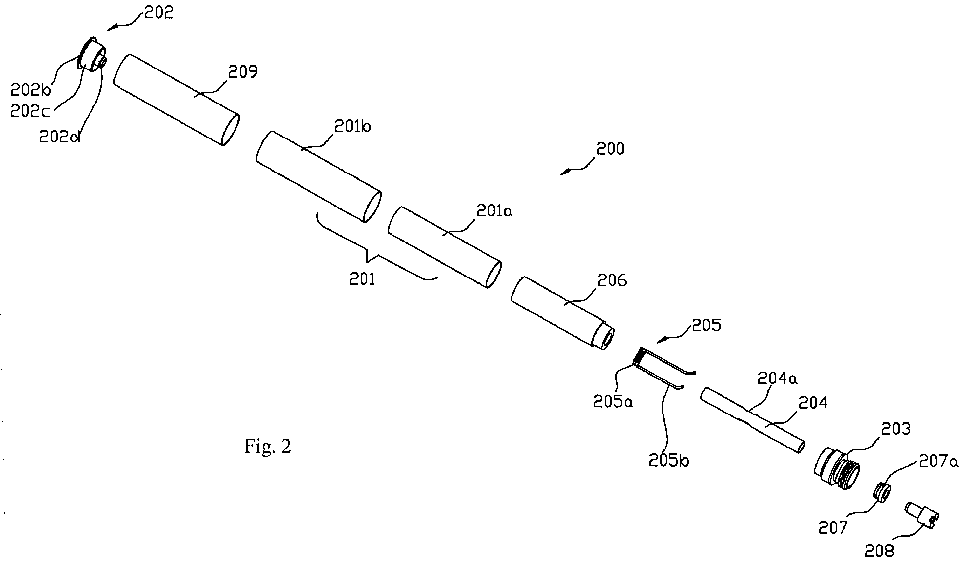

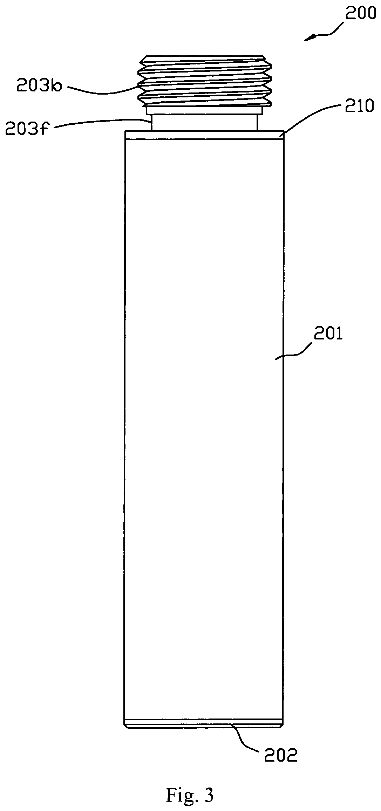

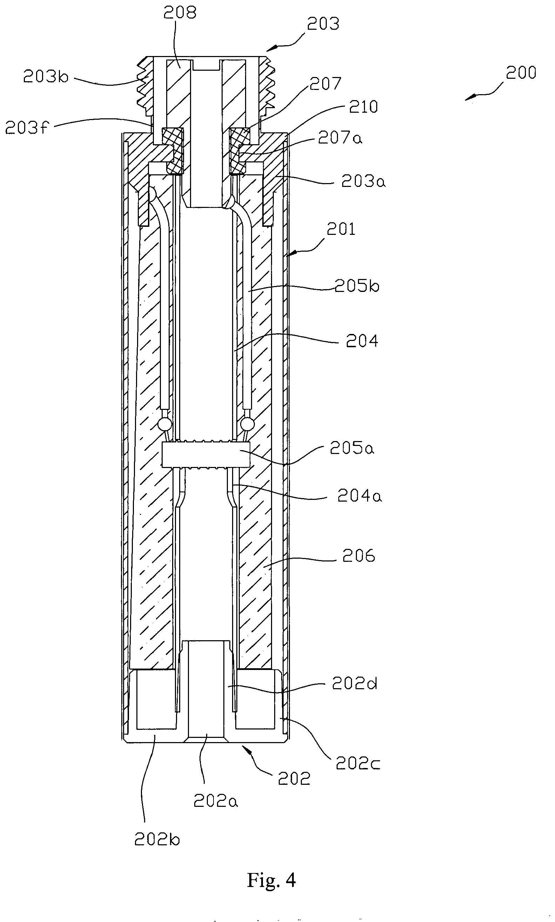

A disposable electronic cigarette of the present invention comprises an atomizer 200 and a battery cartridge 300 connected with each other. Fig. 2 is a schematic explosive view showing the atomizer 200 of the present invention, Fig.3 is a schematic diagram showing the atomizer 200 of the present invention, and Fig.4 is a sectional view of the atomizer 200 shown in Fig. 3. As shown in Figs. 2-4, the atomizer 200 comprises a protective sleeve 201, a suction nozzle cap 202 fixed on a first end of the protective sleeve 201, and a battery cartridge connector 203 fixed on a second end of the protective sleeve 201. The protective sleeve 201 is in cylindric shape. A vent pipe 204 is arranged in the protective sleeve 201, a heating component 205 is arranged on the vent pipe 204 immovably, and an oil-storage cotton 206 coats outside the heating component 205 and the vent pipe 204.

The suction nozzle cap 202 comprises a cover plate 202b with a center hole 202a, and an outer cylinder wall 202c extending perpendicularly from the cover plate 202b, and an inner cylinder wall 202d extending perpendicularly from the periphery of the center hole 202a. The outer cylinder wall 202c and the inner cylinder wall 202d extend in the same direction. The outer cylinder wall 202c is fixed in the first end of the protective sleeve 201 by interference fit.

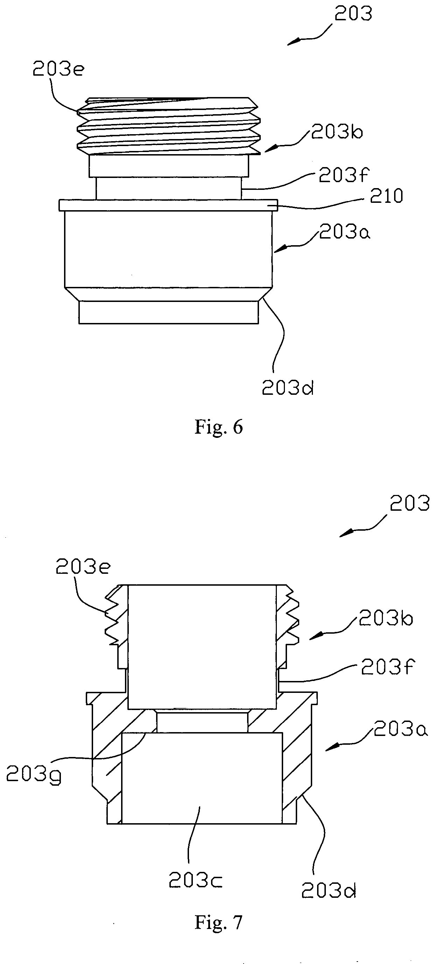

Fig. 6 is a schematic diagram showing a battery cartridge connector of the present invention, and Fig. 7 is a sectional view showing the battery cartridge connector of the present invention. As shown in Figs. 6 and 7, the battery cartridge connector 203 comprises a protective sleeve connecting part 203a and a battery cartridge connecting part 203b connected with each other. The battery cartridge connector 203 has a center hole 203c extending along an axis of the protective sleeve 201. The protective sleeve connecting part 203a is fixed in the second end of the protective sleeve 201 by interference fit. A chamfer 203d is formed on one end of the protective sleeve connecting part 203a, which is far away from the battery cartridge connecting part 203b, so as to assist inserting into the protective sleeve 201. The battery cartridge connecting part 203b is exposed out of the protective sleeve 201. An external thread 203e for connecting the battery cartridge 230 is formed on the battery cartridge connecting part 203b.

A groove 203f is formed on a joint between the protective sleeve connecting part 203a and the battery cartridge connecting part 203b, so that the battery cartridge connector 203 will not be disassembled easily after being fixed to the protective sleeve 201. The groove 203f is formed by cutting the outer surface 203h of the battery cartridge connector 203. The peripheral of the cross section of the battery cartridge connector 203 at the groove 203f in a direction perpendicular to an axis of the protective sleeve 201 is in quadrate, circular, triangular, cone, trapezoidal or wavy shape.

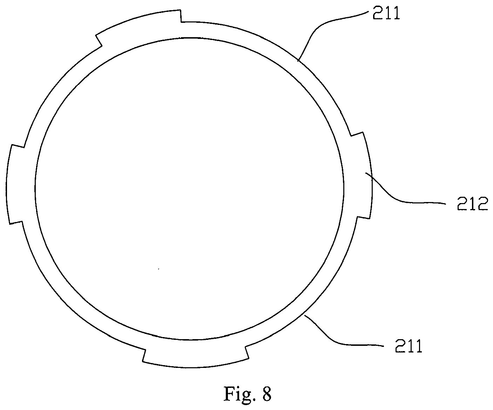

As shown in Fig. 8, the peripheral of the cross section of the battery cartridge connector 203 at the groove 203f in the direction perpendicular to the axis of the protective sleeve 201 may be other irregular shape, for example, comprising multiple recesses 211 arranged alternately. These recesses 211 are positioned uniformly or non-uniformly on a same circumference around the axis of the protective sleeve 201. A bulge 212 is formed between every two adjacent recesses 211. The depths of the recesses 211 may be the same as or different from each other. Similarly, the heights of the bulges 212 may be the same as or different from each other.

The depth of the groove 203f is configured that the battery cartridge connector 203 has a wall thickness of 0.05-0.2mm at the groove 203f. The wall is so thin that the battery cartridge connector 203 will be broken at the groove203f when a force is a applied on the battery cartridge connector 203 in the direction perpendicular to the protective sleeve 201 to bend the battery cartridge connector 203, and the battery cartridge connector 203 will hence not be disassembled. Thus, the disassembly-proof object is achieved. If the thickness of the wall at the groove 203f is less than 0.05mm, it renders difficult to process the battery cartridge connector 203 and the battery cartridge connector 203 will be not in sufficient strength; and If the thickness of the wall at the groove 203f is more than 0.2mm, it will be difficult to break the battery cartridge connector 203 when a force is applied to bend it.

In the embodiment as shown in Fig. 8, the battery cartridge connector 203 has a wall thickness of 0.05-0.1mm at the recesses 211 and a wall thickness of 0.1-0.2mm at the bulges 212.

A flange 210 is formed on the out surface of the protective sleeve connecting part 203a. The flange 210 is formed on one end of the protective sleeve connecting part 203a, closed to the groove 203f. When the protective sleeve connecting part 203a is fixed in the second end of the protective sleeve 201, the flange 210 abuts against the end surface of the second end of the protective sleeve 201 so as to limit the position of the battery cartridge connector 203 and facilitate the assembly.

A first protrudent ring 203g extending along the radial direction of the protective sleeve 201 is formed in the protective sleeve connecting part 203a. A first insulating ring 207 is fixed in the battery cartridge connector 203. The first insulating ring 207 is in shape of circular ring and a first retaining groove 207a is formed on a peripheral surface of the first insulating ring. The whole first insulating ring 207 is made of elastic material, and the first retaining groove 207a is engaged with the first protrudent ring 203g so as to fix the first insulating ring 207 into the battery cartridge connector 203. A first electrode 208 is fixed in the first insulating ring 207 by interference fit. The first electrode 208 is electrically connected with the heating component 205.

As shown in Figs. 2-4, one end of the vent pipe 204 sleeves outside the inner cylinder wall 202d of the suction nozzle cap 202, and the other end abuts against the first insulating ring 207 so that the vent pipe 204 is retained in position.

Two opposite vent holes 204a are formed on the vent pipe 204 and go through its peripheral wall. The heating component 205 is arranged on the vent pipe 204 immovably. The heating component 205 comprises a heater strip body 205a and extending legs 205b extending from two ends of the heating wire body 205a. The heating wire body 205a passes through the two vent holes 204a, and the extending legs 205b clamp on the outer surface of the vent pipe 204 so that the heating component 205 is fixed.

The oil-storage cotton 206 is in cylindric shape substantially and sleeves the heating component 205 and the vent pipe 204. One end of the oil-storage cotton 206 is abutted against an end of the outer cylinder wall 202c of the suction nozzle cap 202, and the other end is sleeved by the protective sleeve connecting part 203a of the battery cartridge connector 203 such that the oil-storage cotton 206 is fixed. A gap is formed between the protective sleeve 201 and the oil-storage cotton 206.

The protective sleeve 201 may comprises an inner protective sleeve 201a and an outer protective sleeve 201b. The inner protective sleeve 201a is made of material such as metal, china, etc.; and the outer protective sleeve 201b is sleeved on the outer protective sleeve 201b and is made of material such as metal, plastic, wood, paper, etc.. A sticker 209 may be arranged on the outer surface of the protective sleeve 201.

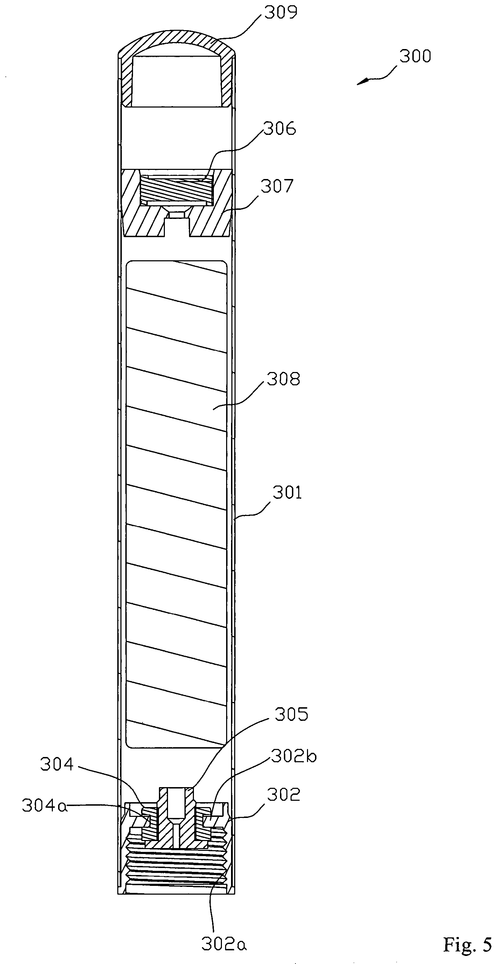

Fig. 5 is a sectional view showing a battery cartridge 300 of the present invention. As shown in Fig. 5, the battery cartridge 300 comprises a cylindric battery sleeve 301. An atomizer connector 302 is arranged in a first end of the battery sleeve 301. The atomizer connector 302 is in cylindric shape substantially and is fixed in the battery sleeve 301 by means such as interference fit, thread and the like. An internal thread 302a for engaging with the external thread 203e on the battery cartridge connector 203 is formed in the atomizer connector 302, so as to connect the atomizer 200 and the battery cartridge 300.

A second protrudent ring 302b extending along the radial direction of the battery sleeve 301 is formed in the atomizer connector 302. A second insulating ring 304 is fixed in the atomizer connector 302. The second insulating ring 304 is in cylindric shape, and a second retaining groove 304a is formed on the peripheral surface of the second insulating ring 304. The whole second insulating ring 304 is made of elastic material. The second retaining groove 304a on the second insulating ring 304 engages with the second protrudent ring 302b so as to fix the second insulating ring 304 into the atomizer connector 302. A second electrode 305 is fixed in the second insulating ring 305 by interference fit. The first electrode 208 is electrically connected with the second electrode 305 when the atomizer 200 and the battery cartridge 300 are connected with each other.

A controller 306 and a controller seat 307 for supporting the controller 306 are arranged in the battery sleeve 301. A battery 308 is arranged between the controller seat 307 and the atomizer connector 302 and is electrically connected with the controller 306 and the second electrode 305 via electrical wires. A light cap 309 is fixed in the second end of the battery sleeve 301.

When the smoker smokes via the suction nozzle cap 202 during use, the controller 306 senses the change of the airflow, connects the battery 308 and the second electrode 305, and further transfers the current flow to the heating component 205 via the first electrode 208 so as to atomize the cigarette liquid in the oil-storage cotton 206. Thus, a large amount of smoke is formed and provided for the smoker's inhalation.

The atomizer 200 can be disassembled and replaced by a new atomizer 200 when the disposable electronic cigarette is used up. Since the groove 203f is formed on the battery cartridge connector 203 of the atomizer 200, the wall at the groove 203f is so thin that the battery cartridge connector 203 will be broken at the groove 203f when a force is applied on the battery cartridge connector 201 in the direction perpendicular to the protective sleeve 201 to bend the battery cartridge connector 203. In other words, the battery cartridge connector 203 will not be disassembled, and the disassembly-proof object is hence achieved. The unqualified cigarette oil can be effectively prevented from being added into the atomizer 200 of the disposable electronic cigarette and reuse of the atomizer is avoided.

The embodiments described above are only some preferred embodiments of the present invention, which are not used to limit the present invention. Any modification, equivalent substitution, and improvement within the principle should be included in the protecting scope of the present invention as defined by the appended claims.

## Claims

1. An disassembly-proof atomizer, comprising a protective sleeve (200), a suction nozzle cap (202) fixed on a first end of the protective sleeve (200), and a battery cartridge connector (203) fixed on a second end of the protective sleeve (200); **characterized in that** a vent pipe (204), a heating component (205) and an oil-storage cotton (206) which coats the outside of the vent pipe (204) and the heating component (205) are arranged in the protective sleeve (200), the battery cartridge connector (203) comprises a protective sleeve connecting part (203a) and a battery cartridge connecting part (203b) connected with each other, a center hole (203c) extending along an axis of the protective sleeve (200) is defined in the battery cartridge connector (203), and a groove (203f) formed by cutting an outer surface (203h) of the battery cartridge connector (203) is defined on a joint between the protective sleeve connecting part (203a) and the battery cartridge connecting part (203b);
an outer peripheral surface of the groove (203f) in a direction perpendicular to an axis of the protective sleeve (200) comprises multiple recesses (211) arranged alternately, and a bulge (212) is formed between every two adjacent recesses (211);
the recesses (211) are positioned uniformly on a same circumference around the axis of the protective sleeve (200); depths of the recesses (211) are the same, and height of each bulge (212) is same with each other;
the battery cartridge connector (203) has a wall thickness of 0.05 mm to 0.1mm at the recesses (211) and a wall thickness of 0.1 mm to 0.2mm at the bulge (212); and
the battery cartridge connector (203) is broken at the groove (203f) when a force is applied on the battery cartridge connector (203) at a direction perpendicular to the protective sleeve (200) to bend the battery cartridge connector (203).

2. The disassembly-proof atomizer of claim 1, **characterized in that** the protective sleeve connecting part (203a) is fixed in the protective sleeve (200) by interference fit.

3. The disassembly-proof atomizer of claim 1, **characterized in that** a chamfer (203d) for assisting inserting into the protective sleeve (200) is formed on one end of protective sleeve connecting part (203a), which is away from the battery cartridge connecting part (203b).

4. The disassembly-proof atomizer of claim 1, **characterized in that** an external thread (203e) is formed on the battery cartridge connecting part (203b).

5. The disassembly-proof atomizer of claim 1, **characterized in that** the disassembly-proof atomizer (200) further comprises a first insulating ring (207) fixed in the battery cartridge connector (203) and a first electrode (208) fixed in the first insulating ring (207), the first insulating ring (207) is made of elastic material; a first retaining groove (207a) is formed on a peripheral surface of the first insulating ring (207), a first protrudent ring (203g) extending along a radial direction of the protective sleeve (200) is formed in the protective sleeve connecting part (203a), and the first retaining groove (207a) is engaged with the first protrudent ring (203g).

6. The disassembly-proof atomizer of claim 1, **characterized in that** the suction nozzle cap (202) comprises a cover plate (202b) with a center hole (202a), an outer cylinder wall (202c) extending perpendicularly from the cover plate (202b), and an inner cylinder wall (202d) circling the center hole (202a) and extending perpendicularly from the cover plate (202b); the outer cylinder wall (202c) is fixed in the first end of the protective sleeve (200) through interference fit.

7. The disassembly-proof atomizer of claim 6, **characterized in that** the oil-storage cotton (206) is in cylindric shape, with one end abutted against an end of the outer cylinder wall (202c) of the suction nozzle cap (202) and the other end sleeved by the protective sleeve (200) connecting part of the battery cartridge connector (203).

8. An electronic cigarette, comprising a battery cartridge and the disassembly-proof atomizer of any one of claims 1-7, **characterized in that** the battery cartridge (300) and the disassembly-proof atomizer (200) connect with each other.

## Patentansprüche

1. Zerlegungssicherer Atomizer, umfassend eine Schutzhülse (200), eine Saugmundstückkappe (202), die an einem ersten Ende der Schutzhülse (200) angebracht ist, und einen Akkuanschluss (203), der an einem zweiten Ende der Schutzhülse (200) angebracht ist, **dadurch gekennzeichnet, dass** ein Entlüftungsröhrchen (204), ein Heizelement (205) und eine Ölspeicherwatte (206), die das Äußere des Entlüftungsröhrchen (204) und das Heizelement (205) umhüllt, in der Schutzhülse (200) angeordnet sind, der Akkuanschluss (203) ein Schutzhülsenanschlussteil (203a) und ein Akkuanschlussteil (203b) umfasst, die miteinander verbunden sind, ein Mittelloch (203c), das entlang einer Achse der Schutzhülse (200) verläuft, in dem Akkuanschluss (203) ausgebildet ist, und eine Nut (203f), die durch Schneiden einer Außenfläche (203h) des Akkuanschlusses (203) hergestellt ist, an einer Verbindungstelle zwischen dem Schutzhülsenanschlussteil (203a) und dem Akkuanschlussteil (203b) ausgebildet ist;
eine äußere Umfangsfläche der Nut (203f) in einer Richtung senkrecht zu einer Achse der Schutzhülse (200) mehrere Vertiefungen (211) umfasst, welche abwechselnd angeordnet sind, und jeweils zwischen zwei benachbarten Vertiefungen (211) eine Ausbuchtung (212) ausgebildet ist;
die Vertiefungen (211) gleichmäßig an einem gleichem Umfang um die Achse der Schutzhülse (200) herum positioniert sind; die Tiefen der Vertiefungen (211) gleich sind, und jede Ausbuchtung (212) dieselbe Höhe hat;
der Akkuanschluss (203) an den Vertiefungen (211) eine Wandstärke von 0,05 mm bis 0,1 mm hat und an der Ausbuchtung eine Wandstärke von 0,1 mm bis 0,2 mm hat; und
der Akkuanschluss (203) an der Nut (203f) bricht, wenn eine Kraft auf den Akkuanschluss (203) in einer Richtung senkrecht zur Schutzhülse (200) ausgeübt wird, um den Akkuanschluss (203) zu biegen.

2. Zerlegungssicherer Atomizer nach Anspruch 1, **dadurch gekennzeichnet, dass** das Schutzhülsenanschlussteil (203a) durch Presspassung in der Schutzhülse (200) befestigt ist.

3. Zerlegungssicherer Atomizer nach Anspruch 1, **dadurch gekennzeichnet, dass** eine Abschrägung (203d) zur Erleichterung der Einführung in die Schutzhülse (200) an einem von dem Akkuanschlussteil (203b) entfernten Ende des Schutzhülsenanschlussteils (203a) ausgebildet ist.

4. Zerlegungssicherer Atomizer nach Anspruch 1, **dadurch gekennzeichnet, dass** ein Außengewinde (203e) an dem Akkuanschlussteil (203b) ausgebildet ist.

5. Zerlegungssicherer Atomizer nach Anspruch 1, **dadurch gekennzeichnet, dass** der nichtzerlegbare Atomizer (200) des Weiteren einen ersten Isolierring (207) umfasst, der in dem Akkuanschluss (203) befestigt ist, und eine erste Elektrode (208), die in dem ersten Isolierring (207) befestigt ist, wobei der erste Isolierring (207) aus elastischem Material besteht; eine erste Haltenut (207a) an einer peripheren Oberfläche des ersten Isolierrings (207) ausgebildet ist, ein erster vorspringender Ring (203g), der entlang einer radialen Richtung der Schutzhülse (200) verläuft, in dem Schutzhülsenanschlussteil (203a) ausgebildet ist, und die erste Haltenut (207a) mit dem ersten vorspringenden Ring (203g) in Eingriff steht.

6. Zerlegungssicherer Atomizer nach Anspruch 1, **dadurch gekennzeichnet, dass** die Saugmundstückkappe (202) eine Abdeckplatte (202b) mit einem Mittelloch (202a), eine äußere Zylinderwand (202c), die senkrecht von der Abdeckplatte (202b) weg verläuft, und eine innere Zylinderwand (202d), die das Mittelloch (202a) kreisförmig umgibt und senkrecht von der Abdeckplatte (202b) weg verläuft, umfasst; wobei die äußere Zylinderwand (202c) durch Presspassung in dem ersten Ende der Schutzhülse (200) befestigt ist.

7. Zerlegungssicherer Atomizer nach Anspruch 6, **dadurch gekennzeichnet, dass** die Ölspeicherwatte (206) zylinderförmig ist, wobei ein Ende an einem Ende der äußeren Zylinderwand (202c) der Saugmundstückkappe (202) anliegt und das andere Ende von dem Schutzhülsen- (200) Anschlussteil des Akkuanschlusses (203) umgeben ist.

8. Elektronische Zigarette, umfassend einen Akku und den zerlegungssicheren Atomizer nach einem der Ansprüche 1-7, **dadurch gekennzeichnet, dass** der Akku (300) und der zerlegungssichere Atomizer (200) miteinander verbunden sind.

## Revendications

1. Atomiseur indémontable comprenant un manchon de protection (200), un capuchon de buse d'aspiration (202) fixé sur une première extrémité du manchon de protection (200), et un connecteur de cartouche de batterie (203) fixé sur une seconde extrémité du manchon de protection (200) ; **caractérisé en ce qu'**un tuyau d'évent (204), un composant de chauffage (205) et un coton de stockage d'huile (206) qui recouvre l'extérieur du tuyau d'évent (204) et le composant de chauffage (205) sont agencés dans le manchon de protection (200), le connecteur de cartouche de batterie (203) comprend une partie de raccordement de manchon de protection (203a) et une partie de raccordement de cartouche de batterie (203b) raccordées entre elles, un trou central (203c) s'étendant le long d'un axe du manchon de protection (200) est défini dans le connecteur de cartouche de batterie (203), et une rainure (203f) formée en coupant une surface externe (203h) du connecteur de cartouche de batterie (203) est définie sur un joint entre la partie de raccordement de manchon de protection (203a) et la partie de raccordement de cartouche de batterie (203b) ;
une surface périphérique externe de la rainure (203f) dans une direction perpendiculaire à un axe du manchon de protection (200) comprend plusieurs évidements (211) agencés de manière alternée, et un renflement (212) est formé entre tous les deux évidements (211) adjacents ;
les évidements (211) sont positionnés, de manière uniforme, sur une même circonférence autour de l'axe du manchon de protection (200) ;
les profondeurs des évidements (211) sont identiques, et la hauteur de chaque renflement (212) est identique aux autres ;
le connecteur de cartouche de batterie (203) a une épaisseur de paroi de 0,05 mm à 0,1 mm au niveau des évidements (211) et une épaisseur de paroi de 0,1 mm à 0,2 mm au niveau du renflement (212) ; et
le connecteur de cartouche de batterie (203) est cassé au niveau de la rainure (203f) lorsqu'une force est appliquée sur le connecteur de cartouche de batterie (203) dans une direction perpendiculaire au manchon de protection (200) pour plier le connecteur de cartouche de batterie (203).

2. Atomiseur indémontable selon la revendication 1, **caractérisé en ce que** la partie de raccordement de manchon de protection (203a) est fixée dans le manchon de protection (200) par ajustement par serrage.

3. Atomiseur indémontable selon la revendication 1, **caractérisé en ce qu'**un chanfrein (203d) pour aider à l'insertion dans le manchon de protection (200), est formé sur une extrémité de la partie de raccordement de manchon de protection (203a), qui est à distance de la partie de raccordement de cartouche de batterie (203b).

4. Atomiseur indémontable selon la revendication 1, **caractérisé en ce qu'**un filetage externe (203e) est formé sur la partie de raccordement de cartouche de batterie (203b).

5. Atomiseur indémontable selon la revendication 1, **caractérisé en ce que** l'atomiseur indémontable (200) comprend en outre une première bague isolante (207) fixée dans le connecteur de cartouche de batterie (203) et une première électrode (208) fixée dans la première bague isolante (207), la première bague isolante (207) est réalisée à partir d'un matériau élastique ; une première rainure de retenue (207a) est formée sur une surface périphérique de la première bague isolante (207), une première bague en saillie (203g) s'étendant le long d'une direction radiale du manchon de protection (200) est formée dans la partie de raccordement de manchon de protection (203a), et la première rainure de retenue (207a) est mise en prise avec la première bague en saillie (203g).

6. Atomiseur indémontable selon la revendication 1, **caractérisé en ce que** le capuchon de buse d'aspiration (202) comprend une plaque de couvercle (202b) avec un trou central (202a), une paroi cylindrique externe (202c) s'étendant perpendiculairement à partir de la plaque de couvercle (202b), et une paroi cylindrique interne (202d) encerclant le trou central (202a) et s'étendant perpendiculairement à partir de la plaque de couvercle (202b) ; la paroi cylindrique externe (202c) est fixée dans la première extrémité du manchon de protection (200) par le biais d'un ajustement par serrage.

7. Atomiseur indémontable selon la revendication 6, **caractérisé en ce que** le coton de stockage d'huile (206) est de forme cylindrique, avec une extrémité en butée contre une extrémité de la paroi cylindrique externe (202c) du capuchon de buse d'aspiration (202) et l'autre extrémité emmanchée par la partie de raccordement de manchon de protection (200) du connecteur de cartouche de batterie (203).

8. Cigarette électronique comprenant une cartouche de batterie et l'atomiseur indémontable selon l'une quelconque des revendications 1 à 7, **caractérisé en ce que** la cartouche de batterie (300) et l'atomiseur indémontable (200) se raccordent l'un par rapport à l'autre.
